(19) Europäisches Patentamt
European Patent Office
Office européen des brevets

(11) **EP 3 302 231 B1**

(12) **EUROPÄISCHE PATENTSCHRIFT**

(45) Veröffentlichungstag und Bekanntmachung des Hinweises auf die Patenterteilung:
**28.10.2020   Patentblatt 2020/44**

(21) Anmeldenummer: **16727314.3**

(22) Anmeldetag: **12.05.2016**

(51) Int Cl.:
*A61B 5/00* (2006.01)          *A61B 5/021* (2006.01)
*A61B 5/024* (2006.01)

(86) Internationale Anmeldenummer:
**PCT/EP2016/060735**

(87) Internationale Veröffentlichungsnummer:
**WO 2016/192952 (08.12.2016 Gazette 2016/49)**

(54) **VERFAHREN SOWIE VORRICHTUNG ZUR ERMITTLUNG DES VERLAUFS DES BLUTDRUCKS**

METHOD AND DEVICE FOR ASCERTAINING A BLOOD PRESSURE CURVE

PROCÉDÉ ET DISPOSITIF PERMETTANT DE DÉTERMINER L'ALLURE DE LA PRESSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.05.2015   DE 102015108518**

(43) Veröffentlichungstag der Anmeldung:
**11.04.2018   Patentblatt 2018/15**

(73) Patentinhaber: **CIS Forschungsinstitut für Mikrosensorik und Photovoltaik GmbH
99099 Erfurt (DE)**

(72) Erfinder: **ORTLEPP, Hans-Georg
99192 Apfelstädt (DE)**

(74) Vertreter: **Engel, Christoph Klaus
PATENTSCHUTZengel
Marktplatz 6
98527 Suhl (DE)**

(56) Entgegenhaltungen:
**US-A1- 2010 160 798     US-B1- 6 616 613**

- **SACHIKO HOMMA ET AL: "RELATIONSHIP BETWEEN ACCELERATED PLETHYSMOGRAM, BLOOD PRESSURE AND ARTERIOLAR ELASTICITY", TAIRYOKU KAGAKU, Bd. 41, Nr. 1, 1. Januar 1992 (1992-01-01), Seiten 98-107, XP055294422, JP ISSN: 0039-906X, DOI: 10.7600/jspfsm1949.41.98**

**Beschreibung**

Die vorliegende Erfindung betrifft ein Verfahren nach Anspruch 1 sowie eine Vorrichtung nach Anspruch 6 zur Ermittlung des zeitabhängigen Verlaufs des Blutdrucks im menschlichen oder tierischen Blutkreislauf.

Für die Einschätzung von Herz-Kreislauf-Risiken spielen Messdaten zu Gefäßsteifigkeit und zentralem Blutdruck eine immer größere Rolle. Etablierte medizinische Diagnosesysteme (SphygmoCor, Complior, Arteriograph) verwenden piezoelektrische, tonometrische oder oszillometrische Messverfahren, um aus der im Kreislauf auftretenden Blutdruckwelle solche Daten zu gewinnen. Der Zeitverlauf des Blutdrucks in einer peripheren Arterie kann durch solche Messverfahren so detailliert aufgelöst werden, dass die Blutdruckfunktion (Druck über die Zeit) sicher in einzelne Teilkomponenten separiert werden kann, auch wenn diese sich teilweise überlappen. Üblicherweise wird die erste Teilkomponente als direkte Welle und die zweite als an der Verzweigung in die beiden großen Beckenarterien reflektierte Welle interpretiert. Diagnostisch relevant sind dann die Höhen der beiden Komponenten und deren zeitliche Differenz. Aus der Zeitdifferenz und der (doppelten) Länge der Aorta kann die Pulswellengeschwindigkeit berechnet werden, die von Blutdruck und Gefäßzustand abhängt.

In verschiedenen Publikationen wurden die Korrelationen zwischen Blutdruck und bestimmten Merkmalen des photoplethysmografisch aufgezeichneten Pulses untersucht. Betrachtet wurden entweder die Zeitdifferenz zwischen der sogenannten R-Zacke eines zusätzlich erfassten EKG und dem Startpunkt der Pulswelle oder ohne EKG bestimmte Formmerkmale der Pulswelle allein.

Unter einem Photoplethysmogramm (PPG) versteht man allgemein ein optisch erhaltenes Plethysmogramm, also die Messung einer Volumenänderung eines Organs. In Bezug auf die vorliegende Erfindung wird durch Aufzeichnung eines Photoplethysmogramms die Volumenänderung an Blutgefäßen ermittelt, die abhängig ist von der im Kreislauf auftretenden Blutdruckwelle. Photoplethysmografiewerte können beispielsweise von Pulsoximetern erfasst werden, die anhand der veränderten Lichtabsorption in durchblutetem peripherem Gewebe einen volumenabhängigen Messwert liefern.

Aus der DE 10 2008 002 747 A1 ist beispielsweise ein Pulsoximeter in Form eines Ohrsensors bekannt. Der Ohrsensor dient der Überwachung mindestens einer physiologischen Messgröße mittels einer nicht-invasiven Messung im Ohrkanal. Dazu umfasst der Ohrsensor mehrere optoelektronische Komponenten, die in einem Gehäuse angeordnet sind, welches in den Ohrkanal einsetzbar ist, wobei die mehreren optoelektronischen Komponenten am Gehäuseumfang verteilt angeordnet sind.

In der US 2013/0253341 A1 sind eine Vorrichtung und ein Verfahren zur nicht-invasiven kontinuierlichen Blutdruckbestimmung beschrieben. Dazu wird die Datenverarbeitung in einem üblichen photoplethysmographischen Messsystem erweitert, um kontinuierlich den Blutdruck nicht-invasiv bestimmen zu können. Allerdings fällt auf, dass die photoplethysmographische Pulswelle wesentlich glatter ist als die periphere Blutdruckwelle, die nach den oben erwähnten Verfahren aufgenommen wurde. Deshalb können in der photoplethysmographisch ermittelten Pulswelle viel weniger Details unterschieden werden.

Insbesondere ist mit den vorbekannten photoplethysmographisch arbeitenden Methoden keine Separation in direkte und reflektierte Teilkomponenten möglich. Blutdruckänderungen lassen sich nur anhand von Änderungen relativ breit verschmierter Formmerkmale bestimmen. Da diese Merkmale aber auch von anderen veränderlichen physiologischen Einflussfaktoren abhängen, ist eine regelmäßige Kalibrierung mit einem Blutdruck-Referenzmesssystem notwendig.

In der US 2014/0012147 sind eine Vorrichtung und ein Verfahren zur kontinuierlichen nicht-invasiven Blutdruckmessung beschrieben, die eine automatischen Rekalibrierung ermöglichen sollen. Dort ist auch auf eine Zeitdauer $\Delta T$ zwischen einem ersten und einem zweiten Maximum im Signalverlauf verwiesen, die aber in keinerlei Beziehung zu der oben erwähnten Pulswellenlaufzeit gebracht werden konnte.

Die US 6,616,613 B1 zeigt eine Vorrichtung und ein Verfahren zur Überwachung physiologischer Signale, wie der Blutvolumenkontur. Dazu wird ein Photoplethysmografie-Sensor an einem Körperteil eines Nutzers positioniert. Aus den elektrischen Signalen des Sensors werden physiologische Parameter bestimmt, welche nachfolgend verarbeitet werden. Nonpulsatile und langsam pulsierende Signale werden aus der Blutvolumenkontur herausgefiltert. Charakteristika der aortalen reflektierten Wellenkontur des Nutzers werden von einer Volumenkontur extrahiert, wobei die Volumenkontur auszuwählen ist aus der Blutvolumenkontur und der gefilterten Blutvolumenpulskontur. Die Charakteristika der aortalen reflektierten Wellenkontur des Nutzers wird teilweise aus der vierten Ableitung der Volumenkontur bestimmt. Die physiologischen Parameter werden dem Nutzer angezeigt.

Die US 2010/160798 A1 offenbart auch ein Verfahren und eine Vorrichtung zur Bestimmung des Blutdrucks, wobei die PPG-Kurve und deren 1. und 2. Ableitungen für die Erkennung von Referenzpunkten eingesetzt werden.

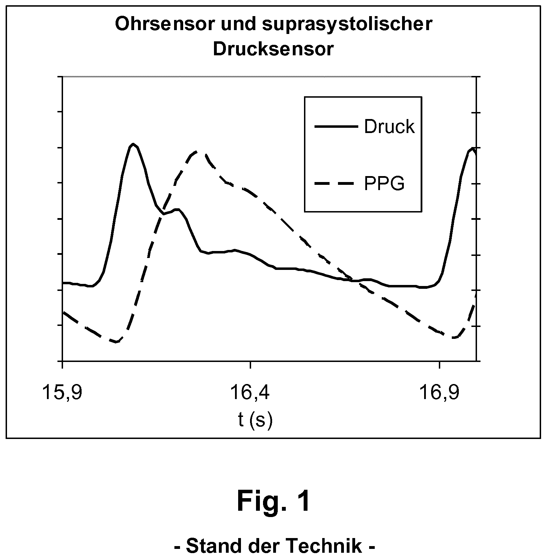

Grundsätzlich ist festzustellen, dass sich bekannte Verfahren zur Aufzeichnung einer Blutdruckwelle nur unter Klinikbedingungen und/oder nur kurzzeitig anwenden lassen. Die sich bei Verwendung herkömmlicher Messmethoden ergebenden Unterschiede im Signalverlauf sind in der beigefügten Fig. 1 veranschaulicht. Dort zeigt die durchgezogene Linie den typischen Druckverlauf einer peripheren Blutdruckwelle, wie sie mit einem suprasystolischen Drucksensor aufgezeichnet wird. Die gestrichelte Linie zeigt stattdessen das am selben Organismus und zur selben Zeit mit einem nicht-invasiv arbeitenden Ohrsensor aufgezeichnete Photoplethysmogramm (PPG). Aus der Darstellung ist ersichtlich, dass die ersten beiden Maxima in der peripheren Druckwelle (durchgezogene Kurve) deutlich ausgeprägt sind und der oben beschriebenen direkten und reflektierten Komponente zugeordnet werden können. Die Zeitdifferenz zwischen den Maxima lässt sich sicher bestimmen und als doppelte Pulswellenlaufzeit interpretieren. Das dritte Maximum in der peripheren Druckwelle stammt von der dikroten Welle. Demgegenüber hebt sich im Photoplethysmogramm (gestrichelte Kurve) nur die dikrote Welle als unterscheidbare Komponente ab. Aus dem Photoplethysmogramm kann mit Methoden des Standes der Technik insbesondere kein Zeitintervall sicher bestimmt werden, aus welchem die Pulswellenlaufzeit bestimmbar wäre.

Die Aufgabe der vorliegenden Erfindung besteht daher darin, ein Verfahren sowie eine Vorrichtung anzugeben, mit denen aus einem Photoplethysmogramm bzw. photoplethysmografisch gewonnenen Messwerten der zeitabhängige Verlauf des Blutdrucks einfach und sicher bestimmt werden kann, d. h. die periphere Blutdruckwelle bestimmbar ist. Diese Aufgabe wird durch ein Verfahren gemäß dem beigefügten Anspruch 1 sowie eine Vorrichtung nach Anspruch 6 gelöst.

Zur Ermittlung des zeitabhängigen Verlaufs des Blutdrucks werden erfindungsgemäß in einem ersten Schritt nicht-invasiv, nämlich mit einem photoplethysmografisch arbeitenden Sensor zeit- und volumenabhängige Durchblutungswerte an einem geeigneten gut durchbluteten Gewebeabschnitt als Photoplethysmografiewerte *P(t)* erfasst. Damit wird faktisch ein Photoplethysmogramm als vorgefilterte Zeitreihe aufgezeichnet. Dies kann mit mit bekannten Mitteln bzw. Sensoren erfolgen, soweit diese ein ausreichend gutes Signal-Stör-Verhältnis besitzen.

Vorzugsweise erfolgt diese Erfassung der Photoplethysmografiewerte *P(t)* am Ohr, beispielsweise am Ohrläppchen oder am äußeren Gehörgang, vorzugsweise am Tragus, mit einem Ohrsensor. Andere Sensoren und Messstellen sind aber ebenfalls geeignet.

In einem zweiten Schritt erfolgt eine Transformation der Photoplethysmografiewerte *P(t)* in Blutdruckwerte *B(t)*. Überraschend hat sich gezeigt, dass dafür die Summe jeweils aus einem Photoplethysmografiewert *P(t),* seiner ersten und zweiten zeitlichen Ableitung gebildet werden muss, wobei alle drei Summanden mit jeweils einem vorbestimmten Koeffizienten beaufschlagt werden. Die Bestimmung der Koeffizienten wird weiter unten näher beschrieben. Mathematisch lässt sich die auszuführende Transformation durch die folgende Transformationsformel beschreiben:

$$B(t) = k0 \cdot P(t) + k1 \cdot P'(t) + k2 \cdot P''(t),$$

mit

der ersten Ableitung $P'(t) = \dfrac{dP}{dt}$

der zweiten Ableitung $P''(t) = \dfrac{d^2 P}{dt^2}$

vorbestimmten Koeffizienten *k0, k1, k2;*

In diesem zweiten Schritt des erfindungsgemäßen Verfahrens erfolgt somit eine lineare Transformation des photoplethysmografischen Pulses in ein Abbild der peripheren Blutdruckwelle.

Schließlich werden in einem abschließenden Schritt die transformierten Blutdruckwerte *B(t)* als zeitabhängige Blutdruckwerte ausgegeben, beispielsweise an eine Anzeige- und/oder Speichereinheit. Es kann sich eine weitere Auswertung und Ableitung der diagnostischen Information nach bekannten Verfahren und Regeln der Verarbeitung von Daten aus Blutdruckwellen-Messungen anschließen.

Eine erfindungsgemäße Vorrichtung zur Ermittlung des zeitabhängigen Verlaufs des Blutdrucks, insbesondere im menschlichen Blutkreislauf, ist so konfiguriert, dass sie die Ausführung des zuvor beschriebenen Verfahrens gestattet. Eine erfindungsgemäße Vorrichtung umfasst dazu einen photoplethysmografischen Sensor, der nicht-invasiv zeit- und volumenabhängige Durchblutungswerte an einem peripheren Blutgefäß als Photoplethysmografiewerte *P(t)* erfasst. Weiterhin ist eine Datenverarbeitungseinheit vorgesehen, welche die Photoplethysmografiewerte *P(t)* in Blutdruckwerte *B(t)* transformiert, wozu insbesondere die oben bereits benannte Transformationsvorschrift implementiert ist. Diese Implementierung kann durch Verwendung von Signalprozessoren und/oder eine Softwareimplementierung erfolgen. Schließlich umfasst die Vorrichtung eine Ausgabe- und Speichereinheit, welche die transformierten Blutdruckwerte *B(t)* als zeitabhängige Blutdruckwerte zumindest temporär speichert und an eine nachgeordnete externe oder interne Anzeige- und/oder Speichereinheit übergibt.

Die Koeffizienten *k0, k1, k2;* sind abhängig von den spezifischen physiologischen Einflussfaktoren. Sie lassen sich leicht durch eine Referenzmessung bestimmen, in welcher die periphere Blutdruckwelle herkömmlich aufgezeichnet und mit der erfindungsgemäß bestimmten verglichen wird. Die einmal gefundenen Koeffizienten können für alle weiteren Messungen unter denselben oder vergleichbaren Bedingungen unverändert benutzt werden. Bei hohen Genauigkeitsanforderungen können die Koeffizienten für jeden einzelnen Patienten als persönliche Werte ermittelt werden. Mit geeigneten Feldmessungen lassen sich aber auch allgemeingültige Koeffizienten für bestimmte Patientengruppen finden.

Zum besseren Verständnis der Erfindung werden nachfolgend insbesondere die Einzelheiten und Abwandlungsmöglichkeiten des Schrittes der Transformation erläutert. Die lineare Transformation kann gemäß einer bevorzugten Ausführungsform durch Faltung mit einem geeigneten Korrelator erfolgen, der aus Differenzenquotienten zusammengesetzt ist.

Dazu werden aus den nicht-invasiv gewonnenen Messwerten digitalisierte Photoplethysmografiewerte $P_i$ mit $N$ Messpunkten und $i = 1 \ldots N$ erzeugt, im Zeitraster $\Delta t$. Die oben genannte allgemeine Formel für die Transformation der Photoplethysmografiewerte in die Blutdruckwerte lässt sich bei Anwendung der Zeitreihendarstellung für die Blutdruckwelle $B_i$, gültig für $i = 2 \ldots N\text{-}1$, wie folgt darstellen:

$$B_i = k0 \cdot P_i + k1 \cdot P'_i + k2 \cdot P''_i$$

Aus den vorhandenen diskreten Messwerten werden die für die Transformation benötigten Ableitungen besonders einfach als Differenzenquotienten gebildet:

$$P'_i = \frac{P_{i+1} - P_{i-1}}{2 \cdot \Delta t} \quad \text{und} \quad P''_i = \frac{P_{i-1} - 2P_i + P_{i+1}}{(\Delta t)^2}$$

Setzt man die Differenzenquotienten in die Transformationsformel ein, ergibt sich:

$$B_i = k0 \cdot P_i + \frac{k1}{2 \cdot \Delta t}(P_{i+1} - P_{i-1}) + \frac{k2}{(\Delta t)^2}(P_{i-1} - 2 \cdot P_i + P_{i+1})$$

Diese Formel kann nach $P_{i-1}$, $P_i$ und $P_{i+1}$ umsortiert werden, um ein FIR-Transformationsfilter (Filter mit endlicher Impulsantwort) zu erhalten, welches beschrieben ist durch:

$$B_i = G_{-1} \cdot P_{i-1} + G_0 \cdot P_i + G_1 \cdot P_{i+1} \,,$$

mit
konstanten Gewichtsfaktoren:

$$G_{-1} = \frac{-k1}{2 \cdot \Delta t} + \frac{k2}{(\Delta t)^2} \,, \qquad G_0 = k0 - \frac{2 \cdot k2}{(\Delta t)^2} \,, \qquad G_1 = \frac{k1}{2 \cdot \Delta t} + \frac{k2}{(\Delta t)^2} \,.$$

Ein wesentlicher Vorteil dieser Berechnungsmethode besteht vor allem darin, dass für jede Wertebestimmung $B_i$ nicht die rechnerisch aufwendige Ermittlung der ersten und zweiten Ableitung erfolgen muss. Vielmehr genügt es, diese aufwendigen Berechnungen einmalig bei der Ermittlung der Gewichtsfaktoren $G_{-1}$, $G_0$ und $G_1$ vorzunehmen. Die Gewichtsfaktoren können dann für alle weiteren Transformationsschritte angewendet werden.

Bevorzugt können in diesem Fall für die Realisierung der erfindungsgemäßen Vorrichtung in die Datenverarbeitungseinheit ein FIR-Filter implementiert werden.

Gemäß einer bevorzugten Ausführungsform erfolgt eine Signalvorfilterung, deren Aufgabe darin besteht, die gemessenen Rohwerte $PR_i$ in vorgefilterte Werte $P_i$ zu überführen. Dies kann besonders bevorzugt mit Hilfe eines FIR-Tiefpasses mit $2k+1$ Koeffizienten $TP_j$, $j = -k \ldots k$ erfolgen.

Um die weiter unten gezeigte Zusammenfassung der Koeffizienten fehlerfrei ausführen zu können, müssen an den Grenzen der Filterfunktion 2 Nullwerte vorgesehen sein: $TP_{-k} = TP_k = 0$. Die Ausführung der Vorfilterung erfolgt nach der Formel:

$$P_i = \sum_{j=-k}^{k} TP_j \cdot PR_{i-j}$$

Gemäß einer nochmals abgewandelten, vorteilhaften Ausführungsform werden die Vorfilterung und die Transformation in einem Rechendurchlauf kombiniert ausgeführt, was sich wie folgt mathematisch beschreiben lässt: In die oben bereits genannte Formel des Transformationsfilters

$$B_i = G_{-1} \cdot P_{i-1} + G_0 \cdot P_i + G_1 \cdot P_{i+1}$$

wird die Vorfilterformel für $P_{i-1}$, $P_i$ und $P_{i+1}$ eingesetzt:

$$B_i = G_{-1} \cdot \sum_{j=-k}^{k} TP_j \cdot PR_{i-j-1} + G_0 \cdot \sum_{j=-k}^{k} TP_j \cdot PR_{i-j} + G_1 \cdot \sum_{j=-k}^{k} TP_j \cdot PR_{i-j+1} \, .$$

Zusammenfassung in eine Summe:

$$B_i = \sum_{j=-k}^{k} TP_j \cdot \left( G_{-1} \cdot PR_{i-j-1} + G_0 \cdot PR_{i-j} + G_1 \cdot PR_{i-j+1} \right).$$

Zusammenfassung der Koeffizienten:

$$KF_j = G_{-1} \cdot TP_{j-1} + G_0 \cdot TP_j + G_1 \cdot TP_{j+1}$$

Damit ergibt sich für den Transformationsfilter:

$$B_i = \sum_{j=-k+1}^{k-1} KF_j \cdot PR_{i-j} \, ,$$

womit Vorfilterung und Transformation in einem kombinierten FIR-Filterdurchlauf mit den $2k$-1 Koeffizienten $KF_j$ erfolgt, mit $j = -k+1...k$-1.

Durch eine besonders bevorzugte Ausführungsform ist die Unterdrückung langwelliger Schwankungen im Druckwellensignal verbessert. In photoplethysmographischen Messkurven schwankt über längere Zeit meistens der Signalgrundpegel, was zu Auswerteproblemen führen kann. Zur Korrektur langwelliger Trends wird üblicherweise ein gleitender Mittelwert über ein geeignetes Zeitintervall gebildet, der von der Messkurve abgezogen wird oder durch den die Messwerte geteilt werden. Für die Erstellung eines Trend-korrigierten Abbilds der Blutdruckkurve kann diese Korrektur entweder vor der oben gezeigten Transformation oder danach erfolgen.

Eine abgewandelte Ausführungsform des Verfahrens zeichnet sich weiterhin dadurch aus, dass zusätzlich zur Ermittlung des zeitabhängigen Verlaufs des Blutdrucks, d. h. der Erstellung des Abbildes der Blutdruckwelle aus dem Photoplethysmogramm, außerdem die Pulswellenlaufzeit aus dem erzeugten Abbild der Blutdruckwelle abgeleitet wird.

Die üblichen Verfahren zur Zerlegung einer nach dem Stand der Technik gemessenen Druckwelle in Teilwellen können dazu auf das erfindungsgemäß erstellte Abbild der Blutdruckwelle angewandt werden. Somit werden die vorbekannten Verfahren auf den erfindungsgemäß ermittelten Werteverlauf angewendet. Vorteilhaft ist die Anpassung der Kurve im Bereich vor dem Einsetzen der Inzisur mit zwei Teilkurven einer geeigneten Modellfunktion, die eine vorzugsweise asymmetrische Glockenkurve beschreibt. Die Zeitdifferenz deren Anfangspunkte oder Maxima wird als Pulswellenlaufzeit interpretiert.

Wenn das Photoplethysmogramm mit einem langzeitlich tragbaren Sensor, vorzugsweise einem Im-Ohr-Sensor, aufgenommen wird, ist damit eine Langzeitüberwachung der Pulswellenlaufzeit möglich. Eine bevorzugte Ausführung der Vorrichtung zeichnet sich somit dadurch aus, dass sie für die Langzeitüberwachung konfiguriert ist, wofür insbesondere ausreichend Speicherkapazität zur Abspeicherung der ermittelten Daten vorgesehen ist.

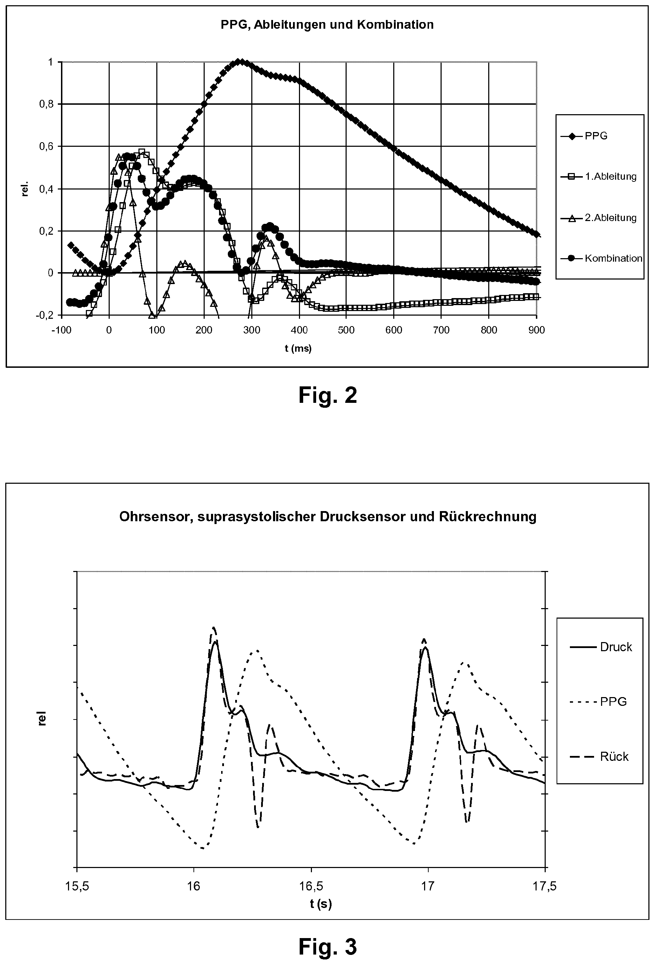

Aus der Physiologie des Herz-Kreislauf-Systems ist bekannt, dass erhöhter Blutdruck das Herz-Kreislauf-Risiko erhöht und dass bei steigendem Blutdruck die Pulswellengeschwindigkeit größer wird. Üblicherweise wird die Pulswellengeschwindigkeit aus der Pulswellenlaufzeit mit einer Umrechnungsformel bestimmt, in die die Körpergröße eingeht. Damit ermöglicht die Überwachung der Pulswellenlaufzeit mit dem erfindungsgemäßen Verfahren auch eine Risikowarnung bei auftretendem Bluthochdruck. Eine demgemäß bevorzugt ausgebildete Vorrichtung zeichnet sich dadurch aus, dass sie eine Signalausgabeeinheit umfasst, welche ein Warnsignal ausgibt, wenn der zeitliche Abstand zwischen dem Auftreten der ersten beiden Peaks in den transformierten Blutdruckwerten *B(t)* einen vorgegebenen zeitlichen Mindestabstand unterschreitet. Da der zweite Peak auf dem Ausläufer des ersten sitzt (vgl. dazu Fig. 3), muss zur Bestimmung eine Komponentenzerlegung erfolgen. Erst nach der erfindungsgemäße Transformation des PPG-Signals werden die beiden Peaks sichtbar und es kann eine Komponentenzerlegung erfolgen.

Weitere Einzelheiten, die dem Verständnis der Erfindung dienen, ergeben sich aus den angefügten Figuren. Es zeigen:

Fig. 1    einen Druckverlauf, gemessen mit einem suprasystolischen Drucksensor, im Vergleich mit einem Photoplethysmogramm, aufgezeichnet mit einem Ohrsensor (Stand der Technik);

Fig. 2    Darstellung einer Transformation eines Photoplethysmogramms (PPG) in ein Abbild einer Blutdruckwelle;

Fig. 3    Vergleich einer direkten Druckmessung mit der mit Hilfe des erfindungsgemäßen Verfahrens aus dem Photoplethysmogramm erzeugten Abbildung der Blutdruckwelle.

Die in Fig. 1 gezeigten Kurven und deren Bedeutung wurden bereits oben mit Bezug auf den Stand der Technik erläutert.

In Fig. 2 kann der Ablauf einer erfindungsgemäß durchzuführenden Transformation eines Photoplethysmogramms (PPG) in ein Abbild einer Blutdruckwelle anhand der dargestellten Kurven nachvollzogen werden. Die Messdaten wurden mit einem Ohr-Sensor aufgenommen, mit einer Datenrate von 100 Samples/s. Dargestellt sind der Verlauf des mit dem Sensor aufgenommenen Photoplethysmogramms (PPG), die berechnete erste und zweite Ableitung, sowie der sich ergebende Verlauf des Blutdrucks als Kombination gemäß der oben erläuterten Formeln.

Fig. 3 zeigt einen Vergleich einer direkten Druckmessung durch suprasystolische Druckwellenmessung (durchgehende Linie) gegenüber der mit Hilfe des erfindungsgemäßen Verfahrens aus dem Photoplethysmogramm (gepunktete Linie) erzeugten Abbildung der Blutdruckwelle (gestrichelte Linie). Die hohe Übereinstimmung zwischen direkter Druckmessung und dem erfindungsgemäße bestimmten Verlauf der Druckkurve ist gut ersichtlich.

**Patentansprüche**

1.    Verfahren zur Ermittlung des zeitabhängigen Verlaufs des Blutdrucks, folgende Schritte umfassend:

- nicht-invasives Erfassen von zeit- und volumenabhängigen Durchblutungswerten an einem gut durchbluteten Gewebeabschnitt mithilfe eines photoplethysmografischen Sensors als Photoplethysmografiewerte *P(t)*;

- Transformation der Photoplethysmografiewerte *P(t)* in Blutdruckwerte *B(t)* durch Anwendung der folgenden Transformationsformel:

$$B(t) = k0 \cdot P(t) + k1 \cdot P'(t) + k2 \cdot P''(t),$$

mit

der ersten Ableitung $P'(t) = \dfrac{dP}{dt}$

der zweiten Ableitung $P''(t) = \dfrac{d^2 P}{dt^2}$

vorbestimmten Koeffizienten *k0, k1, k2;*

- Ausgabe der transformierten Blutdruckwerte *B(t)* als zeitabhängige Blutdruckwerte an eine Anzeige- und/oder Speichereinheit.

2. Verfahren nach Anspruch 1, **dadurch gekennzeichnet, dass**

- digitalisierte Photoplethysmografiewerte $P_i$ mit $N$ Messpunkten und $i = 1 ... N$ erzeugt werden,
- die ersten und zweiten Ableitungen gebildet werden als Differenzquotienten

$$P'_i = \frac{P_{i+1} - P_{i-1}}{2 \cdot \Delta t} \quad \text{und} \quad P''_i = \frac{P_{i-1} - 2P_i + P_{i+1}}{(\Delta t)^2}$$

- die Transformationsformel unter Anwendung der Differenzquotienten umgestellt angewendet wird in der Form:

$$B_i = G_{-1} \cdot P_{i-1} + G_0 \cdot P_i + G_1 \cdot P_{i+1}$$

mit konstanten Gewichtsfaktoren:

$$G_{-1} = \frac{-k1}{2 \cdot \Delta t} + \frac{k2}{(\Delta t)^2} \;, \quad G_0 = k0 - \frac{2 \cdot k2}{(\Delta t)^2} \;, \quad G_1 = \frac{k1}{2 \cdot \Delta t} + \frac{k2}{(\Delta t)^2} \; .$$

3. Verfahren nach Anspruch 1 oder 2, **dadurch gekennzeichnet, dass** die Photoplethysmografiewerte $P(t)$ am äußeren Gehörgang, vorzugsweise an der Innenseite des Tragus erfasst werden.

4. Verfahren nach einem der Ansprüche 1 bis 3, **dadurch gekennzeichnet, dass** die Koeffizienten durch eine Kalibrierungsmessung bestimmt werden, entweder für einen einzelnen Patienten oder für eine Gruppe von Patienten.

5. Verfahren nach einem der Ansprüche 1 bis 4, **dadurch gekennzeichnet, dass** innerhalb einer Signalperiode der zeitliche Abstand zwischen dem Auftreten der ersten beiden Peaks im Verlauf der transformierten Blutdruckwerte $B(t)$ ermittelt wird und ein Warnsignal generiert wird, wenn ein vorgegebener zeitlicher Mindestabstand unterschritten wird, um einen kritischen Blutdruck zu signalisieren.

6. Vorrichtung zur Ermittlung des zeitabhängigen Verlaufs des Blutdrucks umfassend:

- einen photoplethysmografischen Sensor, der nicht-invasiv zeit- und volumenabhängige Durchblutungswerte an einem gut durchbluteten Gewebeabschnitt als Photoplethysmografiewerte $P(t)$ erfasst;
- eine Datenverarbeitungseinheit, welche die Photoplethysmografiewerte $P(t)$ in Blutdruckwerte $B(t)$ transformiert unter Ausführung der folgenden Transformationsvorschrift:

$$B(t) = k0 \cdot P(t) + k1 \cdot P'(t) + k2 \cdot P''(t) \, ,$$

mit

der ersten Ableitung $P'(t) = \dfrac{dP}{dt}$

der zweiten Ableitung $P''(t) = \dfrac{d^2 P}{dt^2}$

vorbestimmten Koeffizienten $k0, k1, k2$;

- eine Ausgabe- und Speichereinheit, welche die transformierten Blutdruckwerte $B(t)$ als zeitabhängige Blutdruckwerte zumindest temporär speichert und an eine nachgeordnete externe oder interne Anzeige- und/oder Speichereinheit übergibt.

7. Vorrichtung nach Anspruch 6, **dadurch gekennzeichnet, dass** der photoplethysmografischen Sensor aus der folgenden Gruppe ausgewählt ist:

- ein Ohrsensor, welcher die Photoplethysmografiewerte *P(t)* am äußeren Gehörgang, vorzugsweise an der Innenseite des Tragus erfasst;
- ein Fingerkuppensensor, welcher die Photoplethysmografiewerte *P(t)* an einer Fingerkuppe erfasst.

**8.** Vorrichtung nach Anspruch 6 oder 7, **dadurch gekennzeichnet, dass** in die Datenverarbeitungseinheit ein FIR-Filter implementiert ist.

**9.** Vorrichtung nach einem der Ansprüche 6 bis 8, **dadurch gekennzeichnet, dass** sie eine Signalausgabeeinheit umfasst, welche ein Warnsignal ausgibt, wenn innerhalb einer Signalperiode der zeitliche Abstand zwischen dem Auftreten der ersten beiden Peaks im Verlauf der transformierten Blutdruckwerte *B(t)* einen vorgegebenen zeitlichen Mindestabstand unterschreitet.

**Claims**

**1.** A method for ascertaining the time-dependent curve of the blood pressure including the following steps:

- noninvasive detection of time-dependent and volume-dependent blood flow values, in the form of photoplethysmographic values P(t), in a section of tissue with good blood circulation by means of a photoplethysmographically functioning sensor;
- transformation of the photoplethysmographic values P(t) into blood pressure values B(t) through the use of the following transformation formula:

$$B(t) = k0 \cdot P(t) + k1 \cdot P''(t),$$

with

the first derivate $P'(t) = \frac{dP}{dt}$

the second derivate $P''(t) = \frac{d^2P}{dt^2}$

predetermined coefficients $ko, k1, k2$;

- outputting of the transformed blood pressure values B(t) as time-dependent blood pressure values to a display and/or memory unit.

**2.** The method according to claim 1, wherein

- digitized photoplethysmographic values $P_i$ with N measurement points and i=1...N are generated,
- the first and second derivates are generated as difference quotients:

$$P'_i = \frac{P_{i+1} - P_{i-1}}{2 \cdot \Delta t} \ \text{and} \ P''_i = \frac{P_{i-1} - 2P_i + P_{i+1}}{(\Delta t)^2}$$

- the transformation, converted through the insertion of difference quotients, is used in the form:

$$B_i = G_{-1} \cdot P_{i-1} + G_0 \cdot P_i + G_1 \cdot P_{i+1}$$

with constant weighting factors:

$$G_{-1} = \frac{-k1}{2 \cdot \Delta t} + \frac{k2}{(\Delta t)^2}, \quad G_{-01} = k0 - \frac{2 \cdot k2}{(\Delta t)^2}, \quad G_1 = \frac{k1}{2 \cdot \Delta t} + \frac{k2}{(\Delta t)^2}.$$

**3.** The method according to claim 1 or 2 wherein the photoplethysmographic values P(t) are detected in the outer ear canal, preferably on the inside of the tragus.

4. The method according to one of the claims 1 to 3, wherein the coefficients are established by means of a calibration measurement, either for an individual patient or for a group of patients.

5. The method according to one of the claim 1 to 4, wherein within a signal period the time interval between the occurrence of the first two peaks in the course of the transformed blood pressure values B(t) is determined and a warning signal is generated if a predetermined minimum time interval is undershot in order to signal a critical blood pressure.

6. A device for ascertaining the time-dependent curve of the blood pressure, including:

- a photoplethysmographic sensor, which noninvasively detects time-dependent and volume-dependent blood flow values, in the form of photoplethysmographic values P(t), in a section of tissue with good blood circulation;
- a data processing unit, which transforms the photoplethysmographic values P(t) into blood pressure values B(t), by executing the following transformation instruction:

$$B(t) = k0 \cdot P(t) + k1 \cdot P''(t),$$

with

the first derivate $P'(t) = \frac{dP}{dt}$

the second derivate $P''(t) = \frac{d^2P}{dt^2}$

predetermined coefficients $ko, k1, k2$;

- an output and memory unit, which at least temporarily stores the transformed blood pressure values B(t) in the form of time-dependent blood pressure values and relays them to a subordinate external or internal display and/or memory unit.

7. The device according to claim 6, wherein the photoplethysmographic sensor is selected from the following group:

- an ear sensor, which detects the photoplethysmographic values P(t) in the outer ear canal, preferably on the tragus; and
- a fingertip sensor, which detects the photoplethysmographic values P(t) at a fingertip.

8. The device according to claim 6 or 7, wherein an FIR filter is implemented in the data processing unit.

9. The device according to one of the claims 6 to 8, wherein a signal output unit is configured to issue a warning signal if within a signal period the time interval between the occurrences of the first two peaks in the curve of the transformed blood pressure values B(t) falls below a predetermined minimum time interval.

**Revendications**

1. Procédé de détermination de l'évolution dans le temps de la tension artérielle, comprenant les étapes suivantes :

- détection non invasive de valeurs de tension artérielle dépendant du temps et du volume au niveau d'une section du tissu bien irriguée par le sang au moyen d'un capteur photopléthysmographique sous forme de valeurs de photopléthysmographie P(t) ;
- transformation des valeurs de photopléthysmographie P(t) en valeurs de tension artérielle B(t) par application de la formule de transformation suivante :

$$B(t) = k0 \cdot P(t) + k1 \cdot P'(t) + k2 \cdot P''(t),$$

avec

la première dérivation $P'(t) = \dfrac{dP}{dt}$ ,

la seconde dérivation $P''(t) = \dfrac{d^2P}{dt^2}$ ,

des coefficients prédéfinis k0, k1, k2,

- édition les valeurs de tension artérielle transformées B(t) sous forme de valeurs de tension artérielle dépendant du temps à une unité d'affichage et/ou de mémoire.

2. Procédé selon la revendication 1, **caractérisé en ce que**

- des valeurs numérisées de photopléthysmographie Pi avec N points de mesure et i = 1... N sont générées,
- les premières et secondes dérivations sont calculées sous forme de quotients différentiels

$$P'_i = \frac{P_{i+1} - P_{i-1}}{2 \cdot \Delta t} \quad \text{et} \quad P''_i = \frac{P_{i-1} - 2P_i + P_{i+1}}{(\Delta t)^2}$$

- la formule de transformation est utilisée convertie en appliquant les quotients différentiels sous la forme :

$$B_i = G_{-1} \cdot P_{i-1} + G_0 \cdot P_i + G_1 \cdot P_{i+1}$$

avec des facteurs de poids constants :

$$G_{-1} = \frac{-k1}{2 \cdot \Delta t} + \frac{k2}{(\Delta t)^2} , \quad G_0 = k0 - \frac{2 \cdot k2}{(\Delta t)^2} , \quad G_1 = \frac{k1}{2 \cdot \Delta t} + \frac{k2}{(\Delta t)^2} .$$

3. Procédé selon la revendication 1 ou 2, **caractérisé en ce que** les valeurs de photopléthysmographie P(t) sont détectées au niveau du canal auditif, de préférence au niveau de la face interne du tragus.

4. Procédé selon une des revendications 1 à 3, **caractérisé en ce que** les coefficients sont définis par une mesure d'étalonnage, soit pour un patient individuel, soit pour un groupe de patients.

5. Procédé selon une des revendications 1 à 4, **caractérisé en ce que**, pendant une période de signalisation, l'écart temporel entre la survenance des deux premières crêtes dans l'évolution des valeurs de tension artérielle transformées B(t) est déterminée et un signal d'alarme est généré si un écart temporel minimal n'est pas atteint afin de signaliser une tension artérielle critique.

6. Dispositif de détermination de l'évolution en fonction du temps de la tension artérielle, comprenant :

- un capteur photopléthysmographique qui détecte de manière non invasive des valeurs de tension artérielle dépendant du temps et du volume au niveau d'une section de tissu bien irrigué par le sang sous forme de valeurs de photopléthysmographie P(t) ;
- une unité de traitement de données qui transforme les valeurs de photopléthysmographie P(t) en valeurs de tension artérielle B(t) en exécutant la règle de transformation suivante :

$$B(t) = k0 \cdot P(t) + k1 \cdot P'(t) + k2 \cdot P''(t) ,$$

avec

la première dérivation $P'(t) = \dfrac{dP}{dt}$ ,

la seconde dérivation $P''(t) = \dfrac{d^2 P}{dt^2}$ ,

des coefficients prédéfinis k0, k1, k2,

- une unité d'édition et de mémoire qui sauvegarde du moins temporairement les valeurs de tension artérielle transformées B(t) sous forme de valeurs de tension artérielle dépendant du temps et les transfère à une unité d'affichage et/ou de mémoire externe ou interne installée en aval.

7. Dispositif selon la revendication 6, **caractérisé en ce que** le capteur photopléthysmographique est sélectionné dans le groupe suivant :

- un capteur auriculaire qui détecte les valeurs de photopléthysmographie P(t) au niveau du canal auditif extérieur, de préférence au niveau de la face interne du tragus ;
- un capteur de bout du doigt qui détecte les valeurs de photopléthysmographie P(t) au niveau d'un bout du doigt.

8. Dispositif selon la revendication 6 ou 7, **caractérisé en ce qu'**un filtre FIR est mis en œuvre dans l'unité de traitement de données.

9. Dispositif selon une des revendications 6 à 8, **caractérisé en ce qu'**il comprend une unité d'édition de signaux qui émet un signal d'alarme si, pendant une période de signalisation, l'écart temporel entre la survenance des deux premières crêtes dans l'évolution des valeurs de tension artérielle B(t) n'atteint pas un écart temporel minimal prédéfini.

**Fig. 1**

**- Stand der Technik -**

**Fig. 2**

**Fig. 3**

**IN DER BESCHREIBUNG AUFGEFÜHRTE DOKUMENTE**

**In der Beschreibung aufgeführte Patentdokumente**

- DE 102008002747 A1
- US 20130253341 A1
- US 20140012147 A
- US 6616613 B1
- US 2010160798 A1